# EUROPÄISCHE PATENTSCHRIFT

(11) **EP 0 681 251 B1**
(45) Veröffentlichungstag und Bekanntmachung des Hinweises auf die Patenterteilung: **12.03.2003**
(21) Anmeldenummer: 95105478.2
(22) Anmeldetag: 12.04.1995
(51) Int. Cl.: G06F 19/00, A61B 19/00

(54) **Verfahren zum Betrieb eines Operationsmikroskopes**
Method of operating an operation microscope
Méthode d'utilisation d'un microscope d'opération

(30) Priorität: 07.05.1994 DE 4416229
(43) Veröffentlichungstag der Anmeldung: 08.11.1995
(73) Patentinhaber: Carl Zeiss, 89518 Heidenheim (Brenz) (DE)
(72) Erfinder: Luber, Joachim, D-73457 Essingen-Forst (DE); Mackevics, Arvids, D-73432 Aalen-Waldhausen (DE)

(56) Entgegenhaltungen:
- WO-A-91/18644
- DE-A- 4 304 571
- US-A- 4 722 056
- US-A- 5 135 299
- IEEE TRANSACTIONS ON BIOMEDICAL ENGINEERING, MARCH 1994, USA, Bd. 41, Nr. 3, ISSN 0018-9294, Seiten 284-286, KRIEF B ET AL 'A new approach to man-machine communication for computerized microscopy'
- PROCEEDINGS OF THE ANNUAL INTERNATIONAL CONFERENCE OF THE ENGINEERI IN MEDICINE AND BIOLOGY SOCIETY, PARIS, OCT. 29 - NOV. 1, 1992, Bd. 14 PART 3, 29.Oktober 1992 MORUCCI J P;PLONSEY R; COATRIEUX J L; SWAMY LAXMINATAYAN, Seite 936/937 XP 000480692 OIKARINEN J ET AL 'DESIGN OF VISUALIZATION SYSTEM FOR NEUROSURGICAL WORKSTATION'

## Beschreibung

Die vorliegende Erfindung betrifft ein Verfahren zum Betrieb eines Operationsmikroskopes in der rechnergestützten stereotaktischen Chirurgie. Insbesondere wird hierbei die Nutzung präoperativ generierter Diagnosebilder zur Orientierung während der eigentlichen Operation angestrebt.

Aus der US 4,722,056 ist ein Operationsmikroskop für den Einsatz in der rechnergestützten stereotaktischen Chirurgie bekannt, bei dem während einer Operation die Einspiegelung von präoperativ generierten Diagnosebildern in die Beobachtungs-Strahlengänge des Operationsmikrokopes und damit die Überlagerung von Diagnosebildern und Sehfeldebene für den Beobachter erfolgt. Eine typische Anwendung findet ein derartiges Operationsmikroskop z.B. in der Neurochirurgie.

Zum Betrieb eines Operationsmikroskopes gemäß der US 4,722,056 ist jedoch eine aufwendige Korrelation der Patienten-, Operationsmikroskop- und Bild-Koordinatensysteme erforderlich, um auf jeden Fall zu gewährleisten, daß das der aktuellen Sehfeldebene entsprechende Diagnosebild in die Beobachtungsoptik des Operationsmikroskopes eingespiegelt wird. So muß etwa während der Operation laufend die jeweilige Position des Sehfeldes erfaßt werden, was durch die Positionsbestimmung des Operationsmikroskopes über ein Ultraschallgeber-System und über die aktuell eingestellten optischen Daten wie Schnittweite etc. erfolgt. Insgesamt resultiert damit ein komplexes Gesamtsystem.

Neben der aufwendigen, laufend erforderlichen Korrelation der jeweiligen Koordinatensysteme stellt jedoch die Einspiegelung zugehöriger, präoperativer Diagnosebilder in das Sehfeld bzw. die entsprechende Überlagerung noch nicht die optimale Orientierungs-Information für den operierenden Chirurgen dar. Problematisch ist dabei nämlich die Tatsache, daß entsprechend zur jeweiligen Vergrößerung der Operationsmikroskop-Optik auch die eingespiegelten Diagnosebilder dementsprechend vergrößert werden müssen. Aufgrund der derzeit möglichen Auflösung in präoperativen bildgebenden Diagnoseverfahren ergibt sich nicht unbedingt ein Vorteil für den operierenden Chirurgen, wenn derartige Originalbilder unmittelbar in der passenden Vegrößerung dem Sehfeld überlagert bzw. eingespiegelt werden. Gefordert sind daher weitere Orientierungshilfen für den Chirurgen, der das Operationsfeld durch das Operationsmikroskop betrachtet.

Aufgabe der vorliegenden Erfindung ist es, ein Verfahren zum Betrieb eines Operationsmikroskopes in der rechnergestützten, stereotaktischen Chirurgie zu schaffen, das eine hinreichende Orientierung des operierenden Chirurgen während der Operation gewährleistet und die Informationen aus präoperativ erzeugten Diagnoseverfahren mit möglichst geringem Aufwand zur Orientierungs-Optimierung heranzieht. Ferner sollte es möglich sein, auch intraoperativ gewonnene Daten hierzu einzusetzen.

Diese Aufgabe wird gelöst durch ein Verfahren mit den Merkmalen des Anspruches 1. Vorteilhafte Ausgestaltungen des erfindungsgemäßen Verfahrens sind Gegenstand der Unteransprüche.

Im Gegensatz zum bekannten Verfahren aus der US 4,722,056 entfällt im erfindungsgemäßen Verfahren nunmehr das laufende aufwendige Korrelieren von Sehfeldebene und präoperativ erzeugten Diagnosedaten bzw. den entsprechenden Diagnosebildern während der eigentlichen Operation.

Es wird vielmehr vor der eigentlichen Operation ein definierter Operationsweg vom Chirurgen auf Grundlage der präoperativ generierten Diagnosedaten festgelegt, auf dem er während der Operation z.B. in Richtung Tumor vordringt. Aus den in einer Bild-Datenbank digitalisiert abgelegten Diagnosedaten wählt der Chirurg vor der Operation ihn interessierende Diagnosebilder aus, die zweidimensionalen Schnittbildern entsprechen, die vorteilhafterweise auf dem Operationsweg liegen. Die entsprechend selektierten Diagnosebilder werden anschließend wieder in der Bild-Datenbank abgespeichert. Die Operationsplanung erfolgt dabei an einer zentralen Steuereinheit.

Während der eigentlichen Operation werden die Diagnosebilder gemäß der vorher definierten Reihenfolge wahlweise in die Beobachtungsoptik des Operationsmikroskopes eingespiegelt, ohne jedoch immer eine explizite Zuordnung zur aktuell eingesehenen Sehfeldebene vorzunehmen.

Hinsichtlich der Einspiegelung in die Beobachtungsoptik des Operationsmikroskopes ist dabei die Wahl zwischen einer Überlagerung der Diagnosebilder mit dem gerade betrachteten Sehfeld als auch die bloße Betrachtung der selektierten Diagnosebilder möglich.

Zusammen mit den selektierten Diagnosebildern werden während der Operation vorteilhafterweise bestimmte Lage-Informationen zu den jeweiligen Diagnosebildern in die Beobachtungsoptik mit eingespiegelt, um dem Chirurgen bei der Betrachtung dieser Bilder eine zumindest grobe räumliche Zuordnung zu ermöglichen.

Das Operationsmikroskop ist an einem motorischen Trägersystem befestigt, an dem es definiert in mindestens drei räumlichen Freiheitsgraden positioniert werden kann.

Dem Chirurgen ist es über das erfindungsgemäße Verfahren nunmehr möglich, nicht nur das jeweils aktuelle, zur Sehefeldebene korrespondierende Diagnosebild zu betrachten, vielmehr kann er vor der Operation bereits aus einer vielfältigen Auswahl Diagnosebilder selektieren, die er während der Operation einsehen möchte.

Hierbei ist es dem Operateur ferner möglich, die einmal ausgewählten Diagnosebilder vor der Operation zu bearbeiten oder zu manipulieren, was beispielsweise durch Einzeichnen von bestimmten interessierenden Konturen in den ausgewählten Diagnosebildern erfolgen kann.

Daneben können zusammen mit den ausgewählten Diagnosebildern auch eine Reihe von patientenspezifischen Informationen abgelegt und während der Operation dem Chirurgen zur Unterstützung im Sehfeld des Operationsmikroskopes eingespiegelt dargeboten werden.

Als vorteilhaft erweist sich weiterhin, die vom operierenden Chirurgen wahrgenommenen Bilder inclusive der überlagerten Diagnosebilder auch auf einem separaten Monitor darzustellen, und derart auch dem Assistenzpersonal diese Informationen zur Verfügung zu stellen.

Weitere Vorteile und Einzelheiten des erfindungsgemäßen Verfahrens ergeben sich aus der nachfolgenden Beschreibung von Ausführungsbeispielen des Verfahrens anhand der beiliegenden Figuren.

Dabei zeigt
- Fig. 1: ein Blockschaltbild mit einzelnen Komponenten zur Durchführung des erfindungsgemäßen Verfahrens;
- Fig. 2a: eine mögliche Darstellung eines präoperativ generierten Diagnosebildes auf einem Display, das zur Operationsplanung herangezogen wird;
- Fig. 2b: die Lagen der ausgewählten Diagnosebilder entlang des Operationsweges in der Darstellung von Fig. 2a;
- Fig. 2c: eine schematisierte perspektivische Gesamtansicht der Lagen der ausgewählten Diagnosebilder entlang des Operationsweges aus Fig. 2b;
- Fig. 3a: ein ausgewähltes Diagnosebild, das vor der Operation manipuliert wird;
- Fig. 3b: das manipulierte Diagnosebild aus Fig. 3a, das lediglich noch die markierten Konturen interessierender Anatomiedetails zeigt.

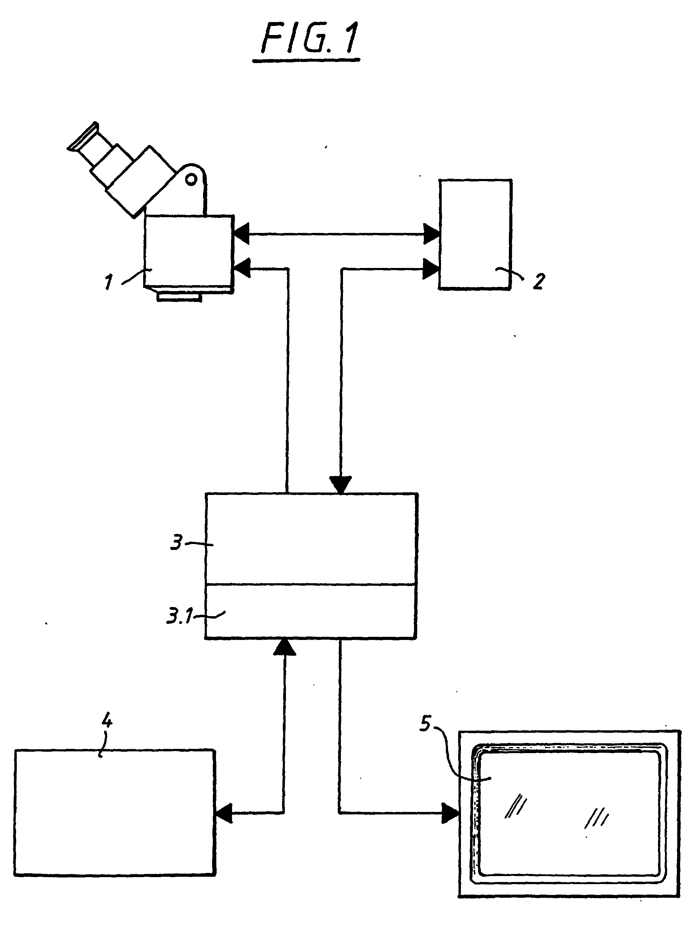

In Figur 1 ist ein Blockschaltbild mit wichtigen erforderlichen Komponenten zur Durchführung des erfindungsgemäßen Verfahrens dargestellt. Vorgesehen ist hierbei ein Operationsmikroskop (1), das an einem - lediglich schematisiert dargestellten - motorischen Trägersystem (2) angeordnet wird. Über das motorische Trägersystem (2) ist ein definiertes, koordinatenmäßiges Positionieren des Operationsmikroskopes (1) in bis zu sechs räumlichen Freiheitsgraden möglich. Das mehrgelenkige, motorische Trägersystem, z.B. dargestellt in der DE 42 02 922 der Anmelderin, umfaßt u.a. den jeweiligen Gelenken zugeordnete Encoder, so daß der zentralen Steuereinheit (3) des Gesamt-Systemes laufend ein Ermitteln der aktuellen koordinatenmäßigen Position des Operationsmikroskopes (1) zusammen mit den bekannten Geometrie-Informationen des Gesamtsystemes möglich ist.

Das Operationsmikroskop weist einen im wesentlichen bekannten optischen Aufbau auf und umfaßt u.a. eine Einspiegelungsvorrichtung, über die eine Einspiegelung anderweitig generierter Bilder über übliche Strahlteiler-Vorrichtungen in mindestens einen Beobachtungs-Strahlengang möglich ist.

Ferner ist ein vorab erstellter Satz präoperativer Diagnosedaten vorgesehen, der in einer Bild-Datenbank (4) abgelegt wird. In der Bild-Datenbank (4) werden die digitalisierten Diagnose-Datensätze abgespeichert, die z.B. über NMR-, CT- oder Röntgen-Untersuchungen vor der eigentlichen Operation generiert wurden. Hierbei können sowohl die Original-Bilder oder aber vom Chirurgen manipulierte Bilder abgespeichert werden. Unter manipulierten Bildern wird hierbei verstanden, daß z.B. Bilder bzw. Datensätze abgelegt wurden, die lediglich eingezeichnete Konturen von bestimmten Anatomie-Details beinhalten. Gleichzeitig ist jedoch die exakte räumliche Position der dergestalt manipulierten Diagnosebilder relativ zum Patienten-Koordinatensystem bekannt.

Die zentrale Steuereinheit (3) übernimmt die laufende Erfassung der Koordinaten des Operationsmikroskopes (1) ebenso wie die Verarbeitung der digitalisierten Diagnose-Datensätze in der Bild-Datenbank (4) und umfaßt hierzu auch eine Eingabeschnittstelle (3.1), über die der Chirurg z.B. die Operationsplanung an der zentralen Steuereinheit (3) vornimmt.

Desweiteren ist ein Display (5) vorgesehen, das die Betrachtung und Manipulation der in der Bild-Datenbank (4) abgelegten Bilder ermöglicht. Ebenso können mit Hilfe des Displays (5) die in das Operationsmikroskop (1) einzuspiegelnden bildmäßigen Informationen in größerem Maßstab vom Assistenzpersonal während der Operation betrachtet werden.

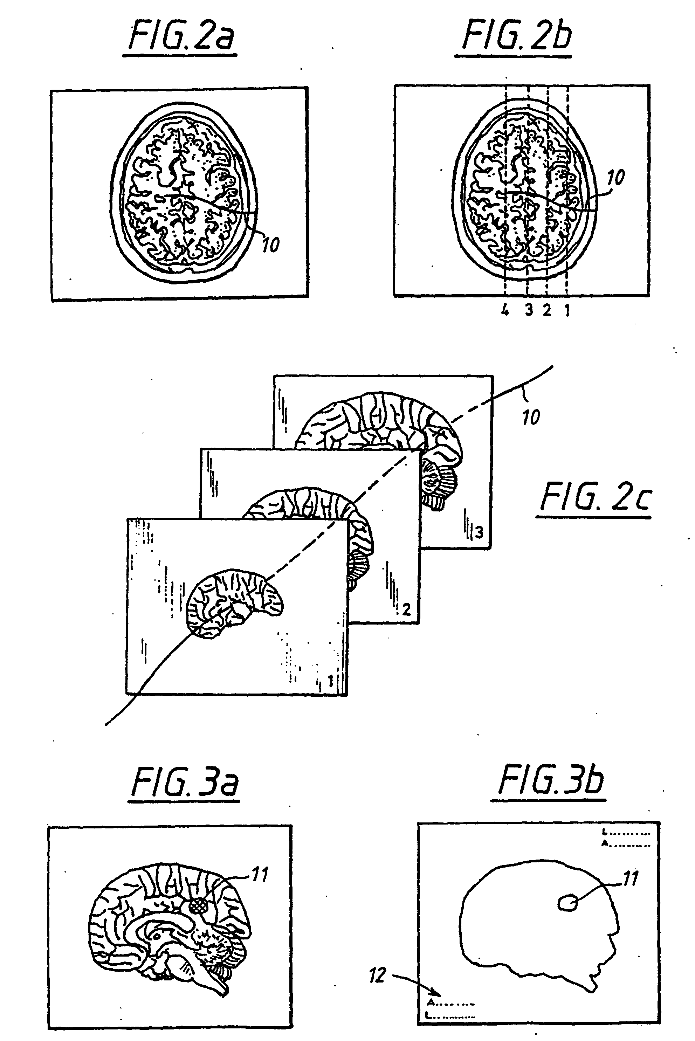

Anhand der Figuren 2a-2c wird nunmehr das erfindungsgemäße Verfahren zum Betrieb eines Operationsmikroskopes näher beschrieben.

Figur 2a zeigt ein präoperativ generiertes Diagnose-Bild, das etwa mit Hilfe einer Computer-Tomographie-Vorrichtung in bekannter Art und Weise gewonnen und in der Bild-Datenbank digitalisiert abgelegt wurde. Dargestellt ist in Figur 2a dabei ein zweidimensionales Schnittbild durch den menschlichen Schädel in einer Ansicht von oben. Neben diesem ausgewählten Bild können Diagnosebilder durch vielfältigste weitere Ebenen im menschlichen Kopf über bekannte Bildverarbeitungsverfahren aus dem erfaßten Diagnosedatensatz rekonstruiert werden.

Bereits vor der eigentlichen Operation wird vom Chirurgen der geplante Operationsweg (10) im Diagnosebild markiert und festgelegt, wie er z.B. erforderlich ist, um zu einem Tumor im Gehirn vordringen zu können. Die mit der Bild-Datenbank verbundene zentrale Steuereinheit erfaßt nach erfolgter Planung den Operationsweg (10) koordinatenmäßig.

Ebenfalls bereits vor der Operation plant der Chirurg nunmehr im erfindungsgemäßen Verfahren, welche Diagnosebilder er während der eigentlichen Operation in das Operationsmikroskop eingespiegelt bekommen möchte. Vier derartige, gewünschte Diagnosebilder (1, .., 4) bzw. deren jeweilige Lage entlang des Operationsweges sind in Figur 2b dargestellt. Vom Chirurgen ist hierbei sowohl die Lage als auch die Orientierung dieser Diagnosebilder relativ zum Operationsweg definiert wählbar. Im dargestellten Ausführungsbeispiel wurden parallel orientierte Diagnosebilder gewählt, die in konstanten Abständen aufeinander folgen.

Noch einmal sei betont, daß sowohl diese Abstände als auch die räumliche Orientierung dieser Diagnosebilder vom Chirurgen vor der Operation frei gewählt werden können.

Eine räumliche Ansicht mit dem geplanten Operationsweg (10) und drei der vier selektierten Diagnosebilder (1, 2, 3) ist in Figur 2c dargestellt. Die Einspiegelung der einmal selektierten Diagnosebilder in die Beobachtungsoptik des eingesetzten Operationsmikroskopes erfolgt nunmehr erfindungsgemäß nicht mehr in definierter Abhängigkeit der aktuellen Sehfeldlage wie etwa in der US 4,722,056, vielmehr ist es dem Chirurgen möglich, die vorher selektierten Bilder jederzeit, d.h. an jeder Stelle im Operationsweg wahlweise abzurufen und einzuspiegeln. Beispielsweise kann nunmehr per Knopfdruck während der Operation der vorab festgelegte Bild-Datensatz mit den vier Diagnosebildern eingespiegelt bzw. eingeblendet werden.

Vorteilhafterweise werden mit den Diagnosebildern dabei gleichzeitig bestimmte Lage-Informationen zu den jeweiligen Diagnosebildern mit eingeblendet, die dem Chirurgen dann die lagemäßige Zuordnung der eingespiegelten Bilder ermöglichen. Derartige Lage-Informationen können z.B. in Form von BildNummern vorgesehen werden, die dann bei etwa konstant-gewähltem Bildabstand jeweils eine Tiefenzuordnung und damit auch die rasche Orientierung entlang des geplanten Operationsweges ermöglichen.

Neben der überlagerten Darstellung von Diagnosebildern und aktuell betrachteter Sehfeldebene ist es in einem alternativen Betriebsmodus des erfindungsgemäßen Verfahrens möglich, lediglich die Diagnosebilder in die Beobachtungsoptik des Operationsmikroskopes einzuspiegeln und auf die überlagerte Darstellung mit dem Sehfeld zu verzichten. Hierzu ist lediglich ein Abblenden der vom Objekt kommenden Strahlengänge mittels bekannter Shutter-Elemente erforderlich. Vorteilhafterweise ist es dem Chirurgen möglich, zwischen der überlagerten Darstellung und der bloßen Betrachtung der eingespiegelten Diagnosebilder während der Operation zu wählen.

Die in den Figuren 2a-2c dargestellten Diagnosebilder entsprechen den Original-Bildern aus den präoperativen Diagnose-Verfahren wie oben beschrieben. Vorteilhaft ist jedoch mitunter, Bilder einzuspiegeln, die lediglich einen reduzierten Informationsgehalt im Vergleich zu den Originalbildern aufweisen. Ein derartig reduzierter Informationsgehalt kann beispielsweise in der Einspiegelung von Konturen bestimmter Anatomiedetails oder dgl. bestehen.

Wie derartige Konturen beispielsweise aus dem ursprünglichen Diagnosebild, das eine definierte räumliche Beziehung mit dem Patienten aufweist, generiert werden, ist in den Figuren 3a und 3b dargestellt. Dort wird z.B. mit Hilfe einer geeigneten Computer-Maus ein Tumor (11) im Original-Diagnosebild graphisch markiert und ein modifiziertes Bild gemäß Fig. 3b erzeugt, das lediglich noch diese Kontur des Tumors (11) zeigt. Lediglich dieses Bild mit dem Tumor-Umriß (11) wird dann in das Sehfeld des Operationsmikroskopes eingespiegelt, während auf die restlichen Anatomie-Informationen bewußt verzichtet wird.

Ebenfalls in Figur 3b dargestellt sind weitere Informationsfelder (12) im eingespiegelten Diagnosebild,- in die sowohl spezielle patientenspezifische Informationen eingetragen werden als auch die oben erwähnten Lage-Informationen zum jeweiligen Diagnose-Bild, die dem Chirurgen während der Operation eine räumliche Zuordnung dieses Bildes ermöglichen. In die Informationsfelder (12) können z.B. der Patientenname, Informationen zum aktuellen Gerätezustand oder aber bestimmte intraoperative Patienten-Informationen wie Blutdruck, Pulsschlag etc. eingeblendet werden, die während der Operation laufend erfaßt werden. Selbstverständlich ist es auch möglich, intraoperative Antworten auf Stimulationsprozesse in geeigneter graphischer Form einzuspiegeln.

Als hierbei einzuspiegelnde Lage-Informationen kommen etwa die Bild-Nummer aus der selektierten Diagnosebild-Auswahl, oder aber der Abstand zum folgenden oder vorhergehenden Bild etc. in Frage.

Desweiteren ist zur Orientierungsoptimierung für den Chirurgen möglich, graphische Informationen dem Sehfeld überlagert einzuspiegeln, die dem Chirurgen andeuten, ob er sich noch auf dem ursprünglich geplanten Operationsweg befindet. Hierfür ist es erforderlich, den gerade betrachteten Punkt koordinatenmäßig zu erfassen und zu vergleichen, ob dieser Punkt noch auf dem tatsächslich geplanten Operationsweg liegt. Das koordinatenmäßige Erfassen dieses Punktes kann dabei etwa mit einem Operationsmikroskop erfolgen, wie es in der DE 41 34 481 der Anmelderin dargestellt ist. Je nachdem, ob sich der Operateur noch auf dem richtigen, geplanten Weg befindet, wird dies über die Steuereinheit anschließend graphisch für den Chirurgen visualisiert. Das kann beispielsweise derart erfolgen, daß eine graphische Markierung in das Sehfeld eingespiegelt werden, die den aktuell betrachteten Punkt darstellt und gleichzeitig eine weitere graphische Markierung eingespiegelt wird, die einem Punkt auf dem tatsächlichen Operationsweg entspricht. Durch manuelles oder motorisches Positionieren des Operationsmikroskopes kann dieser Punkt auf dem Operationsweg wieder angefahren werden. Als graphische Markierungen können etwa Strichkreuze oder dgl. eingesetzt werden.

Während der eigentlichen Operation wird das Operationsmikroskop nunmehr mit Hilfe des motorischen Trägersystems entlang des festgelegten Operationswegs verfahren. Der Chirurg kann dabei sowohl die Verfahrgeschwindigkeit, als auch bestimmte Haltepositionen je nach Anforderung bereits bei der erwähnten Operationsplanung vorab festlegen.

Die Verfahrgeschwindigkeit wird etwa je nach aktuell betrachtetem Sehfeld bzw. in Abhängigkeit des betrachteten Bereiches gewählt. Dies bedeutet, daß etwa in bestimmten interessierenden Regionen des Operationsgebietes ein bedeutend langsameres Verfahren des Operationsmikroskopes erfolgen kann.

Benötigt der Chirurg nunmehr an irgendeiner Stelle des vorher festgelegten Operationsweges die bildmäßigen Informationen aus den präoperativ generierten Diagnosebildern, so kann er jederzeit den vorab selektierten Bild-Datensatz abrufen und in das Sehfeld einspiegeln. Eine Korrelation dieser Bilder mit der aktuellen Sehfeldposition erfolgt dabei nicht, die erforderlichen Lageinformationen erhält der Chirurg aus den zusammen mit dem Bild eingeblendeten Informationen wie Bild-Nummer und den vorab bestimmten Bild-Abständen. Desweiteren ist dabei nunmehr ein "Durchblättern" der kompletten Diagnosebilder-Auswahl möglich, d.h. es resultiert insgesamt eine verbesserte und vielfältigere Nutzung der präoperativen Diagnosedaten während der Operation.

Neben der Möglichkeit über das erfindungsgemäße Verfahren die Operationsplanung vor der eigentlichen Operation vorzunehmen, besteht ferner die Möglichkeit, diese einmal durchgeführte Planung während der Operation zu modifizieren. Wenn etwa der einmal geplante Zielpunkt auf den ursprünglich geplanten Operationsweg angefahren wurde, kann nunmehr das Operationsmikroskop in verschiedenen Blickrichtungen um diesen Ziel-Punkt räumlich geschwenkt werden. Der nunmehr geänderten Blickrichtung zum Ziel-Punkt entsprechen nunmehr auch andere Diagnosebilder aus dem Diagnose-Datensatz. Wie bereits vorab beschrieben, ist es möglich, Diagnosebilder in frei wählbaren Abständen und Orientierungen hierzu auszuwählen. Beim weiteren Verfahren des Operationsmikroskopes entlang des nunmehr modifizierten Operationsweges kann der Chirurg die selektierten Diagnosebilder wie vorab beschrieben wahlweise und unabhängig von der aktuellen Sehfeld-Position in die Beobachtungsoptik des Operationsmikroskopes eingespiegelt betrachten. Es ist somit auch die intraoperative Verwendung des erfindungsgemäßen Verfahrens möglich.

## Patentansprüche

1. Verfahren zum Betrieb eines Operationsmikroskopes in der rechnergestützten stereotaktischen Chirurgie, wobei
- zunächst ein Diagnose-Datensatz über ein bildgebendes, präoperatives Diagnoseverfahren generiert wird und dieser Diagnose-Datensatz in einer Bilddatenbank (4) digitalisiert abgelegt wird,
- anschließend eine Operationsplanung an einer zentralen Steuereinheit (3) mit Hilfe des Diagnose-Datensatzes aus der Bilddatenbank (4) erfolgt und dabei ein Operationsweg (10) festgelegt wird,
- zum derart festgelegten Operationsweg (10) aus dem Diagnose-Datensatz zweidimensionale Diagnosebilder in frei wählbaren Abständen und Orientierungen selektiert werden,
- während der Operation das an einem motorischen Trägersystem (2) angeordnete Operationsmikroskop (1) entlang des festgelegten Operationsweges (10) definiert motorisch verfahren wird und die selektierten Diagnosebilder wahlweise und unabhängig von der aktuellen Sehfeld-Position in die Beobachtungsoptik des Operationsmikroskopes (1) eingespiegelt werden.

2. Verfahren nach Anspruch 1, wobei die generierten Diagnosebilder nach der Festlegung des Operationsweges (10) bearbeitet, anschließend manipuliert abgespeichert und während der Operation manipuliert in die Beobachtungsoptik des Operationsmikroskopes (1) eingespiegelt werden.

3. Verfahren nach Anspruch 2, wobei bei der Manipulation der Diagnosebilder Konturen (11) in die Diagnosebilder eingezeichnet werden, die bestimmten Anatomiedetails entsprechen.

4. Verfahren nach Anspruch 1, wobei zusammen mit den selektierten Diagnosebildern wählbare Lage-Informationen zu jedem Diagnosebild in die Beobachtungsoptik des Operationsmikroskopes (1) eingespiegelt werden, die eine lagemäßige Zuordnung des jeweiligen Diagnosebildes zum geplanten Operationsweg (10) ermöglichen.

5. Verfahren nach Anspruch 4, wobei die Diagnosebilder in wählbaren Abständen zueinander entlang des Operationsweges (10) selektiert werden und der Abstand zum vorigen und/oder folgenden Bild mit dem als Lage-Information während der Operation zusammen mit jedem Diagnosebild eingespiegelt wird.

6. Verfahren nach Anspruch 1, wobei zusammen mit den selektierten Diagnosebildern weitere patientenspezifische Informationen abgelegt und in Informationsfeldern (12) in den Diagnosebildern während der Operation in die Beobachtungsoptik des Operationsmikroskopes (1) eingespiegelt werden.

7. Verfahren nach Anspruch 6, wobei intraoperative Antworten auf Stimulationsprozesse in geeigneter graphischer Form eingespiegelt werden.

8. Verfahren nach mindestens einem der vorangehenden Ansprüche, wobei die Diagnosebilder während der Operation noch auf mindestens einem weiteren Monitor (5) dargestellt werden.

9. Verfahren nach mindestens einem der vorangehenden Ansprüche, wobei während der Operation die Wahl zwischen einer überlagerten Darstellung von aktuell betrachteter Sehfeldbene und selektierten Diagnosebildern und der bloßen Darstellung der Diagnosebilder im Operationsmikroskop (1) möglich ist.

10. Verfahren nach mindestens einem der vorangehenden Ansprüche, wobei die Verfahr-Geschwindigkeit des Operationsmikroskopes (1) entlang des festgelegten Verfahrweges in Abhängigkeit des jeweils betrachteten Ortes vom Operateur vor der Operation an der zentralen Steuereinheit (3) bei der Operationsplanung wählbar ist.

## Claims

1. Method for operating a surgical microscope in computer-assisted stereotactic surgery, in which method
- a set of diagnostic data is firstly generated using an image-forming, pre-operative diagnosis procedure, and this set of diagnostic data is stored in digitized form in an image data bank (4),
- a surgical procedure is then planned at a central control unit (3) with the aid of the set of diagnostic data from the image data bank (4), and a surgical route (10) is established,
- for the surgical route (10) thus established, two-dimensional diagnostic images are selected at freely selectable intervals and orientations from the set of diagnostic data,
- and, during the operation, the surgical microscope (1) arranged on a motor-driven support system (2) is motor-driven along the established surgical route (10) in a defined manner and the selected diagnostic images are reflected into the viewing optics of the surgical microscope (1) alternately and independently of the actual visual field position.

2. Method according to Claim 1, in which, after the surgical route (10) has been established, the generated diagnostic images are processed, then manipulated, stored, and, during the operation, reflected into the viewing optics of the surgical microscope (1) in manipulated form.

3. Method according to Claim 2, in which, during the manipulation of the diagnostic images, contours (11) are introduced into the diagnostic images, which contours (11) correspond to defined anatomical details.

4. Method according to Claim 1, in which, together with the selected diagnostic images, selectable position-information items for each diagnostic image are reflected into the viewing optics of the surgical microscope (1) and permit positional allocation of the respective diagnostic image to the planned surgical route (10).

5. Method according to Claim 4, in which the diagnostic images are selected at selectable intervals from one another along the surgical route (10), and the interval from the previous and/or succeeding image is reflected in as position information during the operation together with each diagnostic image.

6. Method according to Claim 1, in which, together with the selected diagnostic images, further patient-specific information items are stored and, during the operation, are reflected in information fields (12) in the diagnostic images into the viewing optics of the surgical microscope (1).

7. Method according to Claim 6, in which intraoperative responses to stimulation processes are reflected in in suitable graphic form.

8. Method according to at least one of the preceding claims, in which the diagnostic images are also presented on at least one further monitor (5) during the operation.

9. Method according to at least one of the preceding claims, in which it is possible, during the operation, to choose between a superimposed representation of the current visual field plane and selected diagnostic images and simple representation of the diagnostic images in the surgical microscope (1).

10. Method according to at least one of the preceding claims, in which the speed of travel of the surgical microscope (1) along the established path of travel can be chosen as a function of the respectively considered position of the surgeon before the operation at the central control unit (3) during the surgical planning.

## Revendications

1. Procédé pour le fonctionnement d'un microscope d'opération en chirurgie stéréotactique assistée par ordinateur, dans lequel :
- au départ, un jeu de données de diagnostic est généré via une procédure de diagnostic préopératoire produisant des images et ce jeu de données de diagnostic est stocké par voie numérique dans une banque de données d'images (4),
- ensuite, on effectue une planification de l'opération dans une unité de commande centrale (3) à l'aide du jeu de données de diagnostic issu de la banque de données d'images (4) et l'on établit en l'occurrence un chemin opératoire (10),
- on sélectionne, pour le chemin opératoire (10) ainsi établi, parmi le jeu de données de diagnostic, des images bidimensionnelles de diagnostic à des distances et selon des orientations qui peuvent être librement choisies, et
- au cours de l'opération, le microscope d'opération (1) agencé sur un système de support motorisé (2) est déplacé par motorisation de manière définie le long du chemin opératoire établi (10) et les images de diagnostic sélectionnées sont reproduites dans l'optique d'observation du microscope d'opération (1) au choix et indépendamment de la position courante du champ de vision.

2. Procédé selon la revendication 1, dans lequel les images de diagnostic générées sont traitées après établissement du chemin opératoire (10), puis stockées par manipulation et reproduites, par manipulation au cours de l'opération, dans l'optique d'observation du microscope d'opération (1).

3. Procédé selon la revendication 2, dans lequel, lors de la manipulation des images de diagnostic, on dessine dans les images de diagnostic des contours (11) qui correspondent à certains détails anatomiques.

4. Procédé selon la revendication 1, dans lequel on reproduit dans l'optique d'observation du microscope d'opération (1), conjointement avec les images de diagnostic sélectionnées, des informations de position sélectionnables pour chaque image de diagnostic, qui permettent une affectation selon la position de l'image de diagnostic respective au chemin opératoire planifié (10).

5. Procédé selon la revendication 4, dans lequel les images de diagnostic sont sélectionnées à des distances mutuelles sélectionnables le long du chemin opératoire (10) et la distance par rapport à l'image précédente et/ou à l'image suivante est reproduite avec celle des informations de position au cours de l'opération conjointement avec chaque image de diagnostic.

6. Procédé selon la revendication 1, dans lequel d'autres informations spécifiques au patient sont stockées conjointement avec les images de diagnostic sélectionnées et sont reproduites dans des champs d'informations (12) des images de diagnostic au cours de l'opération dans l'optique d'observation du microscope d'opération (1).

7. Procédé selon la revendication 6, dans lequel des réponses intra-opératoires à des processus de stimulation sont reproduites sous une forme graphique appropriée.

8. Procédé selon au moins l'une quelconque des revendications précédentes, dans lequel les images de diagnostic sont représentées au cours de l'opération encore sur au moins un autre moniteur (5).

9. Procédé selon au moins l'une quelconque des revendications précédentes, dans lequel, au cours de l'opération, il est possible de choisir entre une représentation superposée du plan de champ de vision couramment considéré et des images de diagnostic sélectionnées et la simple représentation des images de diagnostic dans le microscope d'opération (1).

10. Procédé selon au moins l'une quelconque des revendications précédentes, dans lequel la vitesse de déplacement du microscope d'opération (1) le long du chemin de déplacement établi peut être choisie par l'opérateur en fonction du lieu respectivement considéré avant l'opération à l'unité de commande centrale (3) lors de la planification de l'opération.
